# EUROPÄISCHE PATENTSCHRIFT

(11) **EP 0 684 025 B1**
(45) Veröffentlichungstag und Bekanntmachung des Hinweises auf die Patenterteilung: **05.01.2000**
(21) Anmeldenummer: 94810226.4
(22) Anmeldetag: 25.04.1994
(51) Int. Cl.: A61F 2/46, A61B 17/56

(54) **Ausziehinstrument für einen Schaft einer Hüftgelenkprothese oder einer entsprechenden Raspel**
Extraction instrument for a stem of a hip prosthesis or a corresponding rasp
Instrument extracteur pour une tige d'une prothése de hanche ou une râpe correspondante

(43) Veröffentlichungstag der Anmeldung: 29.11.1995
(73) Patentinhaber: Sulzer Orthopädie AG, 6340 Baar (CH)
(72) Erfinder: Müller, Stephan, CH-3600 Thun (CH)
(74) Vertreter: Trieblnig, Adolf

(56) Entgegenhaltungen:
- EP-A- 0 408 109
- WO-A-91/06262
- DE-B- 2 101 002
- DE-U- 9 212 846
- FR-A- 2 151 863
- US-A- 3 857 389
- US-A- 4 686 971

## Beschreibung

Die Erfindung betrifft ein Ausziehinstrument entsprechend dem Oberbegriff des Patentanspruchs 1.

Eine Vorrichtung, welche die Merkmale des Oberbegriff von Anspruch 1 aufweist, ist aus der WO-A-91/06262 bekannt.

Ein aus der EP-Patentanmeldung Nr. 0 550 118 bekanntes Ausziehinstrument der genannten Art enthält eine mit einem Griffteil starr verbundene Halterung, welche einen eine Halspartie des auszuziehenden Schafts umgreifenden ringförmigen Ansatz und einen an ihr angelenkten Schwenkhebel umfasst. Der Schwenkhebel ist über einen am Griffteil beweglich angebrachten Verstellteil oder über ein mit dem Griffteil gekoppeltes Gestänge gegen die vom ringförmigen Ansatz umgebene Halspartie verspannbar. Die bekannte Ausführung enthält mehrere aufeinander abzustimmende, bewegliche Teile, die einen entsprechend grossen Arbeits- und Zeitaufwand bei der Herstellung und beim Zusammenbau sowie beim Reinigen und Sterilisieren des Instruments erfordern.

Der Erfindung liegt die Aufgabe zugrunde, ein weiter entwickeltes Instrument der eingangs genannten Gattung in einer gegenüber bisherigen Ausführungen vereinfachten, kompakten Bauweise zu schaffen, welche wenige, robust ausführbare und leicht zu handhabende Bauteile enthält und welche eine starre einfach lösbare Verbindung mit dem Schaft ermöglicht und über welche die für das Aunschlagen des Schaftkörpers erforderlichen. Zugkräfte auf die Halspartie übertragen werden.

Diese Aufgabe wird durch die im kennzeichnenden Teil des Patentanspruchs 1 angegebenen Merkmale gelöst.

Ein Vorteil der erfindungsgemässen Ausführung besteht darin, dass die Halterung den auszuziehenden Schaft der Prothese oder der Raspel in dem den Kopfteil aufnehmenden Endabschnitt der Halspartie, und damit an einer vom Knochengewebe entfernten Stelle erfasst, so dass die in den Schaft einzuleitende Kraft im wesentlichen in diesem Endabschnitt, im Abstand vom Knochengewebe übertragen wird. Entsprechend werden die Teile des Ausziehinstruments vom Knochengewebe ferngehalten, wobei ständig eine ungehinderte Beobachtung des Implantationsbereichs gewährleistet werden kann. Ein weiterer Vorteil der erfindungsgemässen Ausführung besteht darin, dass der auf die Halspartie aufsetzbare Hülsenteil mit dem Kupplungselement eine kompakte, einfach betätigbare Halterung bildet, welche die Anordnung der zu kuppelnden Teile in einem rundum geschlossenen, kappenartigen Gehäuse ermöglicht. Die erfindungsgemässe Vorrichtung zeichnet sich insbesondere aus durch einfach herstellbare und einfach zusammenbaubare Teile, die leicht gereinigt und sterilisiert werden können.

Ausgestaltungen des Erfindungsgegenstandes sind in den abhängigen Patentansprüchen angegeben.

Die Ausführung nach Anspruch 2 ermöglicht eine einfach herstellbare und einfach lösbare kraft- und formschlüssige Verbindung zwischen dem gegen die Halspartie verspannbaren Kupplungselement und dem gegen dieses in Richtung der gemeinsamen Zentrierachse verspannbaren Hülsenteils.

Mit der Ausführung nach Anspruch 3 ist eine besonders kompakte Bauweise der Halterung erzielbar. Das in Form einer Spannhülse ausgeführte Kupplungselement kann aus einem relativ einfach herstellbaren Drehteil gefertigt werden und eine relativ geringe Wandstärke aufweisen.

Die Ausführung nach Anspruch 4 ermöglicht die Ausbildung einer einfach herstellbaren und lösbaren Schnappverbindung zwischen dem konischen Endabschnitt der Halspartie und der auf diese in axialer Richtung aufsteckbaren, am Hülsenteil beweglich verspannbar gehaltenen Spannhülse.

Die Ausführung nach Anspruch 5 gestattet eine von der Arbeitsstellung der Halterung unabhängige Einstellung des Handhabungsteils bzw. eines entsprechenden, z.B. mit unterschiedlichen Handhabungsteilen koppelbaren Anschlussteils und ermöglicht dadurch, innerhalb der durch die anatomischen Verhältnisse gegebenen Grenzen, eine Uebertragung der in den Schaft einzuleitenden Kräfte in unterschiedlichen Winkelstellungen des Handhabungsteils.

Die Ausführung nach Anspruch 6 gestattet die Uebertragung der in den Schaft einzuleitenden Kräfte auf einer Wirkungslinie, die im wesentlichen mit einer Hauptachse des Schaftkörpers zusammenfällt, obwohl die Kräfte an der vom Schaftkörper seitlich abstehenden Halspartie angreifen.

Weitere Einzelheiten und Merkmale ergeben sich aus der folgenden Beschreibung eines in der Zeichnung schematisch dargestellten Ausführungsbeispiels nach der Erfindung. Es zeigen:
- Fig.1: ein erfindungsgemäss ausgebildetes Ausziehinstrument für einen Schaft einer Endoprothese in einer Seitenansicht,
- Fig.2: Teile eines entsprechenden Ausziehinstruments in einer abgewandelten Auführungsform, in einer Teilansicht mit einem axialen Teilschnitt,
- Fig.3: das Ausziehinstrument nach Fig.2 in einer Draufsicht, und
- Fig.4: eine Einzelheit des Ausziehinstruments in einer Draufsicht mit einem Teilschnitt entsprechend der Linie IV - IV in Fig.2.

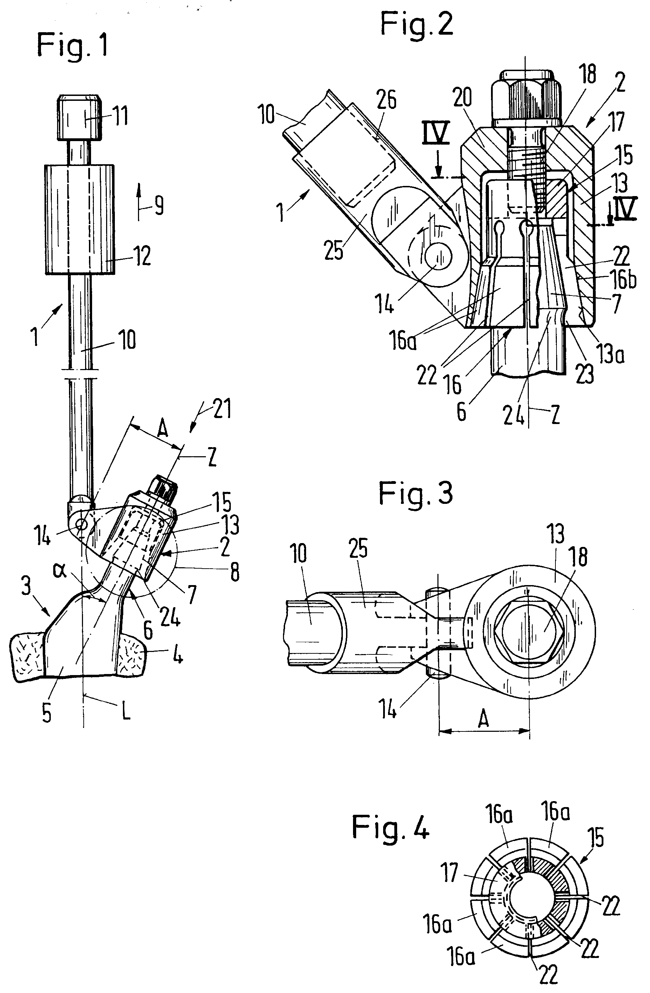

Das Ausziehinstrument nach Fig.1 enthält einen Handhabungsteil 1 und eine mit diesem verbundene Halterung 2, die mit einem Schaft 3 einer Hüftgelenkprothese kraft- und formschlüssig kuppelbar ist. Der Schaft 3 enthält einen in einen Oberschenkelknochen 4 einschlagbaren Schaftkörper 5 und eine von diesem seitlich abstehende Halspartie 6, welche mit einem bezüglich einer Zentrierachse Z konisch sich verjüngenden Endabschnitt 7 ausgeführt ist. Die Zentrierachse Z ist darstellungsgemäss unter einem Winkel α zu einer Längsachse L des Schaftkörpers 5 geneigt. Die Halspartie 6 dient als Aufsteckdorn für eine in Fig.1 strichpunktiert dargestellte künstliche Gelenkkugel 8, die mit einer auf den Endabschnitt 7 passenden konischen Aufnahmebohrung ausgeführt ist. Um eine Anpassung der Einbaulage der zu implantierenden Gelenkkugel 8 und damit des Drehpunkts der Hüftgelenkprothese an die jeweiligen anatomischen Verhältnisse zu ermöglichen, kann in bekannter Weise ein Satz aus mehreren, mit unterschiedlich tiefen Aufnahmebohrungen ausgeführten Gelenkkugeln 8 bereitgestellt werden, aus dem die passende Gelenkkugel ausgewählt und selbsthemmend auf den Endabschnitt 7 aufgesetzt wird.

Anstelle des dargestellten Prothesenschafts 3 kann auch eine in Form und Grösse entsprechende, in den Knochen 4 einschlagbare und zurückziehbare Raspel vorgesehen sein, auf deren Halspartie 7 jeweils eine der Gelenkkugel 8 entsprechende Probierkugel aufgesteckt und probeweise mit einer nicht dargestellten Hüftgelenkpfanne zusammengeführt werden kann, um die endgültige Einbaulage der Gelenkkugel 8 zu bestimmen.

Der Handhabungsteil 1 enthält eine mit der Halterung 2 gelenkig verbundene Führungsstange 10 mit einem an ihrem freien Ende angeordneten Anschlag 11 für einen auf der Führungsstange 10 verschiebbar angebrachten Gleithammer 12. Durch den gegen den Anschlag 11 bewegten Gleithammer 12 kann in bekannter Weise jeweils eine zum Ausziehen des Schafts 3 bestimmte Kraft über die Führungsstange 10 und die Halterung 3 in den Schaftkörper 5 eingeleitet werden, um diesen aus dem Knochengewebe auszuschlagen. Wie insbesondere aus der Fig.2 hervorgeht, enthält die Halterung 2 einen auf die Halspartie 6 aufsetzbaren glockenartigen Hülsenteil 13, der an der Führungsstange 10 um einen Zapfen 14 schwenkbar angelenkt ist, und ein Kupplungselement 15 in Form einer in den Hülsenteil 13 einführbaren und auf den Endabschnitt 7 der Halspartie 6 aufsteckbaren Spannhülse. Das Kupplungselement 15 ist mit einer den Endabschnitt 7 umgebenden Seitenwand 16 und einem über den Endabschnitt 7 vorstehenden Kopfteil 17 ausgeführt, der mittels einer zentralen Spannschraube 18 gegen eine entsprechende Kopfpartie 20 des Hülsenteils 13 verspannbar gehalten ist. Die Seitenwand 16 ist in einem auf den Endabschnitt 7 aufsteckbaren Längenabschnitt mit einer entsprechend konischen Innenfläche ausgeführt und durch in Aufsteckrichtung (Pfeil 21) offene Längsschlitze 22 in mehrere, gemäss Fig.4 in acht, radial bewegliche zungenartige Wandsegmente 16a unterteilt, welche je gegen den Endabschnitt 7 federnd verspannbar sind.

Wie aus der Fig.2 weiter hervorgeht, können die Wandsegmente 16a an ihren freien Enden je mit einer nach innen vorstehenden, wulstartigen Mitnehmerpartie 23 versehen sein. Die Mitnehmerpartien 23 sind so ausgeführt, dass sie beim Aufstecken des Kupplungsteils 15 auf den Endabschnitt 7 in eine an diesen anschliessende, hinterschnittene Einschnürung 24 der Halspartie 6 einrasten und somit in diese Einschnürung 24 einrückbare und ausrückbare Anschlagteile bilden, über welche die für das Ausschlagen des Schaftkörpers 5 erforderlichen Zugkräfte auf die Halspartie 6 übertragen werden.

Gemäss Fig.2 kann die Endpartie der Seitenwand 16 des Kupplungselements 15 mit einer in Aufsteckrichtung (Pfeil 21) divergierenden konischen Aussenfläche 16b ausgeführt sein, die zum Zusammenführen mit einer entsprechend konischen Innenfläche 13a des Hülsenteils 13 bestimmt ist. Die entsprechende Verbindung kann selbsthemmend ausgeführt sein. Das mit dem Hülsenteil 13 lose verbundene Kupplungselement 15 kann auf den Endabschnitt 7 der Halspartie 6 aufgesteckt werden, bis die Mitnehmerpartien 23 in die Einschnürung 24 einrasten, und anschliessend durch Anziehen der Spannschraube 18 gegen die Innenfläche 13a sowie gegen den Endabschnitt 7 verspannt werden, so dass eine in Richtung der Zentrierachse Z starre, einfach lösbare Verbindung zwischen der Halterung 2 und dem Schaft 3 hergestellt wird.

Gemäss Fig.1 kann die am Hülsenteil 13 ausgebildete Anlenkstelle in einem solchen seitlichen Abstand A von der Zentrierachse Z angeordnet sein, dass bei auf die Halspartie 6 aufgesetztem Hülsenteil 13 der Zapfen 14 im wesentlichen in einer Verlängerung der Längsachse L des Schaftkörpers 5 liegt und damit die Wirkungslinie der jeweils in den Schaft 3 einzuleitenden Kraft mit dieser Längsachse L zusammenfällt.

Entsprechend der Darstellung nach den Fig.2 und 3 kann die am Handhabungsteil 1 vorgesehene Anlenkstelle für die Halterung 2 an einem mit der Führungsstange 10 oder einem entsprechenden Griffteil kuppelbaren Anschlussteil 25 ausgebildet sein, welcher darstellungsgemäss eine mit einem Gewinde versehene Kupplungshülse zur Aufnahme eines in diese einschraubbaren Gewindeabschnitts 26 der Führungsstange 10 enthält.

Es sind zahlreiche abgewandelte Ausführungsformen der Erfindung möglich. So kann z.B. der Hülsenteil 13 mit dem Handhabungsteil 1 oder mit dem Anschlussteil 25 starr verbunden sein. Der Hülsenteil 13 und das Kupplungselement 15 können an ihren zusammenführbaren Endpartien mit zylindrischen Führungsflächen ausgeführt sein. Ebenso kann das Kupplungselement 15 mit einer auf einen zylindrischen Endabschnitt einer entsprechenden Halspartie aufsteckbaren zylindrischen Innenfläche ausgeführt sein. Anstelle der dargestellten Spannhülse kann auch eine andere Kupplungsanordnung, z.B. in Form von in einem entsprechenden Hülsenteil angeordneten, gegen die Halspartie 6 verspannbaren losen Mitnehmerelementen, vorgesehen sein. Die erfindungsgemässe Halterung 2 kann ferner auch an einem nicht dargestellten Handhabungsteil einer Vorrichtung zum Einschlagen des Schafts 3 in den Knochen 4 angebracht oder mit einem solchen Handhabungsteil kuppelbar sein.

Zusammenfassend lässt sich die Erfindung wie folgt beschreiben:

Das Ausziehinstrument enthält einen mit einer Halspartie 6 eines auszuziehenden Schafts koppelbaren Hülsenteil 3 und ein Kupplungselement 15 in Form einer auf die Halspartie 6 aufsteckbaren Spannhülse. Die Spannhülse enthält einen Längenabschnitt mit gegen die Halspartie 6 verspannbaren Wandsegmenten 16a und mit in eine Einschnürung 24 der Halspartie 6 einführbaren Mitnehmern 23 sowie einen über die Halspartie 6 vorstehenden Kopfteil 17, der über eine Spannschraube 18 gegen den Hülsenteil 13 verspannbar ist. Das mit dem Hülsenteil 13 lose verbundene Kupplungselement 15 wird auf die Halspartie 6 aufgesteckt und durch Anziehen der Spannschraube 16 gegen den Hülsenteil 13 und gegen die Halspartie 6 verspannt. Entsprechend wird auf einfache Weise eine starre, einfach lösbare Verbindung zwischen dem Ausziehinstrument und dem auszuziehenden Schaft erzielt.

## Patentansprüche

1. Ausziehinstrument für einen Schaft (3) einer Hüftgelenkprothese oder einer entsprechenden Raspel, der bzw. die einen in einen Knochen (4) einschlagbaren zurückziehbaren Schaftkörper (5) und eine mit einem Kopfstück, insbesondere einer künstlichen Gelenkkugel (8), bestückbare Halspartie (6) aufweist, welches Ausziehinstrument ein Handhabungsteil (1) und eine an die Halspartie (6) ansetzbare Halterung (2) aufweist, welche ein auf die Halspartie (6) aufsetzbares Hülsenteil (13) mit einer die Halspartie (6) zumindest teilweise aufnehmenden Bohrung aufweist sowie ein am Hülsenteil (13) abstützbares, gegen die Halspartie (6) verspannbares Kupplungselement (15),
**dadurch gekennzeichnet,**
dass die Halterung (2) einen mit einer Ausnehmung der Halspartie (6) zusammenführbaren Mitnehmer (23) enthält, welcher an einem in die Ausnehmung der Halspartie (6) einrückbaren und ausrückbaren Abschnitt des Kupplungselements (15) ausgebildet ist.

2. Ausziehinstrument nach Anspruch 1, dadurch gekennzeichnet, dass die Bohrung des Hülsenteils (13) und das Kupplungselement (15) mit in Richtung einer gemeinsamen Zentrierachse (Z) zusammenführbaren Führungsabschnitten ausgeführt sind, von denen mindestens einer eine in Aufsetzrichtung des Hülsenteils bezüglich der Zentrierachse divergierende Führungsfläche (16b, 13a) aufweist.

3. Ausziehinstrument nach Anspruch 1 oder 2, dadurch gekennzeichnet, dass das Kupplungselement (15) in Form einer zumindest teilweise in den Hülsenteil (13) einführbaren Spannhülse ausgeführt ist, welche einen auf die Halspartie (6) aufsteckbaren Längenabschnitt aufweist, der durch in Aufsteckrichtung (Pfeil 21) offene Längsschlitze (22) in mehrere radial bewegliche, zungenartige Wandsegmente (16a) unterteilt ist und welche einen über die Halspartie (6) vorstehenden Kopfteil (17) aufweist, der über eine Schraubverbindung (18) mit dem Hülsenteil (13) koppelbar ist.

4. Ausziehinstrument nach Anspruch 3, dadurch gekennzeichnet, dass die Spannhülse mit einer auf einen konischen Endabschnitt (7) der Halspartie (6) aufsetzbaren, entsprechend konischen Innenfläche ausgeführt ist, die in Aufsteckrichtung (Pfeil 21) der Spannhülse durch nach innen vorstehende, einem entsprechend abgesetzten Abschnitt (24) der Halspartie (6) zugeordnete, als Mitnehmer (23) ausgebildete Endpartien der zungenartigen Wandsegmente (16a) begrenzt ist.

5. Ausziehinstrument nach einem der vorangehenden Ansprüche, dadurch gekennzeichnet, dass die Halterung (2) mit dem Handhabungsteil (1) oder einem diesem zugeordneten Anschlussteil (25) um eine Anlenkstelle (14) schwenkbar verbunden ist.

6. Ausziehinstrument nach Anspruch 5, dadurch gekennzeichnet, dass die Anlenkstelle (14) an der Halterung (2) in einem solchen seitlichen Abstand (A) vom Hülsenteil (13) ausgebildet ist, dass sie bei auf die Halspartie (6) aufgesetztem Hülsenteil (13) zumindest annähernd in der Verlängerung einer Längsachse (L) des Schaftkörpers (5) liegt.

## Claims

1. Extraction instrument for a shaft (3) of a hip joint prosthesis or of a corresponding rasp, which has a shaft member (5) which can be tapped into and withdrawn from a bone (4) and a neck portion (6) which can be fitted with a head piece, in particular with an artificial joint ball (8), with the extraction instrument having a handling part (1) and a holder (2) which can be placed on the neck portion (6) and which comprises a sleeve portion (13) mountable on the neck portion (6) and having a bore which at least partly receives the neck portion (6), as well as a coupling element (15) which can be supported at the sleeve portion (13) and clamped against the neck portion (6), characterised in that the holder (2) contains a driver (23) which can be brought together with a recess of the neck portion (6) and which is formed at a section of the coupling element (15) which is engageable into and disengageable from the recess of the neck portion (6).

2. Extraction instrument in accordance with claim 1, characterized in that the bore of the sleeve portion (13) and the coupling element (15) are realised with guide sections which can be brought together in the direction of a common centering axis (Z), and of which at least one has a guide surface (16b, 13a) which diverges relative to the centering axis in the direction of fitment of the sleeve portion.

3. Extraction instrument in accordance with claim 1 or 2, characterized in that the coupling element (15) is realised in the form of a collet which is at least partially insertable into the sleeve portion (13) and which has an elongate section which can be placed onto the neck portion (6), wherein the elongate section is subdivided into a plurality of radially movable tongue-like wall segments (16a) by open longitudinal slots (22) which extend in the direction of fitment (arrow 21) and wherein the collet has a head part (17) which projects beyond the neck portion (6) and which can be coupled to the sleeve portion (13) via a threaded connection (18).

4. Extraction instrument in accordance with claim 3, characterized in that the collet has a conical inner surface which can be placed onto a correspondingly conical end section (7) of the neck portion (6), wherein the conical inner surface is bounded in the direction of fitment (arrow 21) of the collet by inwardly projecting end parts of the tongue-like wall segments (16a), the end parts being formed as drivers (23) and associated with a correspondingly stepped section (24) of the neck portion (6).

5. Extraction instrument in accordance with one of the preceding claims, characterized in that the holder (2) is pivotally connected to the handling part (1), or to a connector part (25) associated with the handling part, about a pivot point (14).

6. Extraction instrument in accordance with claim 5, characterized in that the pivot point (14) at the holder (2) is formed at a lateral distance (A) from the sleeve portion (13) such that, when the sleeve portion (13) is placed on the neck portion (6), the pivot point lies at least approximately in the extension of a longitudinal axis (L) of the shaft member (5).

## Revendications

1. Instrument extracteur pour une tige (3) d'une prothèse d'articulation de la hanche ou d'une râpe correspondante, qui présente un corps de tige (5) pouvant être enfoncé dans et retiré d'un os (4), et une partie de col (6) pouvant être équipée d'une pièce de tête, notamment d'une boule d'articulation artificielle (8), l'instrument extracteur présentant une pièce de manipulation (1) et un logement (2), applicable à la partie de col (6), qui présente une partie de manchon (13) pouvant être placée sur la partie de col (6) avec un perçage recevant au moins partiellement la partie de col (6), ainsi qu'un élément d'accouplement (15) pouvant prendre appui sur la partie de manchon (13), pouvant être tendue contre la partie de col (6), caractérisé en ce que le logement (13) comprend un organe d'entraînement (23) pouvant être réuni avec un évidement de la partie de col (6), qui est réalisé à un tronçon de l'élément d'accouplement (15) pouvant être introduit dans et retiré de l'évidement de la partie de col (6).

2. Instrument extracteur selon la revendication 1, caractérisé en ce que le perçage de la partie de manchon (13) et l'élément d'accouplement (15) sont réalisés avec des tronçons de guidage, pouvant être réunis en direction d'un axe de centrage commun Z, dont au moins un présente une surface de guidage (16b, 13a) divergeant dans la direction d'enfichage de la partie de manchon relativement à l'axe de centrage.

3. Instrument extracteur selon la revendication 1 ou 2, caractérisé en ce que l'élément d'accouplement (15) est réalisé sous forme d'un manchon tendeur pouvant être inséré au moins partiellement dans la partie de manchon (13), qui présente un tronçon longitudinal pouvant être placé sur la partie de col (6), qui est divisé par des fentes longitudinales (22) ouvertes dans la direction d'enfichage (flèche 21) en plusieurs segments de paroi (16a) en forme de languettes, déplaçables radialement et qui présentent une partie de tête (17) faisant saillie sur la partie de col (6), qui peut être accouplée par un assemblage par vissage (18) avec la partie de manchon (13).

4. Instrument extracteur selon la revendication 3, caractérisé en ce que le manchon tendeur est réalisé avec une surface intérieure conique correspondante, pouvant être placée sur un tronçon d'extrémité conique (7) de la partie de col (6), qui est limité dans la direction d'enfichage (flèche 21) du manchon tendeur par des parties d'extrémité des segments de paroi en forme de languettes (16a), faisant saillie vers l'intérieur, associées à un tronçon étagé de manière correspondante (24) de la partie de col (6), réalisées comme organe d'entraînement (23).

5. Instrument extracteur selon l'une des revendications précédentes, caractérisé en ce que le logement (2) est relié d'une manière pivotante autour d'un point d'articulation (14) à la partie de manipulation (1) ou à une partie de raccordement (25) associée à celle-ci.

6. Instrument extracteur selon la revendication 5, caractérisé en ce que le point d'articulation (14) est réalisé au logement (2) à un tel écart latéral (A) de la partie de manchon (13) que, lorsque la parie de manchon (13) est placée sur la partie de col (6), celui-ci se situe au moins approximativement dans le prolongement d'un axe longitudinal (L) du corps de tige (5).
